# EUROPEAN PATENT SPECIFICATION

(11) **EP 2 910 276 B1**
(45) Date of publication and mention of the grant of the patent: **23.06.2021**
(21) Application number: 15156002.6
(22) Date of filing: 20.02.2015
(51) Int. Cl.: A61M 37/00, A61M 35/00

(54) **Skin treatment device**
Hautbehandlungsvorrichtung
Dispositif de traitement de la peau

(30) Priority: 21.02.2014 GB 201403104
(43) Date of publication of application: 26.08.2015
(73) Proprietor: Hilditch, Sharon, Liverpool, Merseyside L1 9AA (GB)
(72) Inventor: Hilditch, Sharon, Liverpool, Merseyside L1 9AA (GB)
(74) Representative: Brand Murray Fuller LLP

(56) References cited:
- CN-A- 102 727 992
- CN-B- 101 670 145
- KR-A- 20080 100 569
- KR-A- 20090 039 073
- KR-A- 20110 106 659
- US-A1- 2011 092 884

## Description

The present invention relates to skin treatment devices and, in particular, but without limitation to, micro needle roller (MNR) devices for transdermal delivery of therapeutic or cosmetic agents with cooled, oxygen-enriched air. The invention is particularly applicable to cosmetic and therapeutic treatments of the skin.

The use of oxygen in cosmetic and therapeutic treatments has been known for many years. It is also well known to apply beauty care products to the skin, usually in the form of a cream or lotion, that is rubbed into the outer layer of the skin, the epidermis. Various techniques have been proposed in the art whereby oxygen is used to then force the cream or lotion through the epidermis by application of high pressure oxygen.

Equally, it is known that various micro needle arrays and dermal rollers can be used in conjunction with creams or lotions for forcing the cosmetic or therapeutic creams or lotions through the punctures in the epidermis caused by the micro needles.

A major drawback of known transdermal delivery of agents is that often a local anaesthetic is required to take away the pain that can be felt during treatment.

What the applicant has recognised is that by supplying cooled pressurised oxygen with the dermal roller, this takes the much of the discomfort away during treatment. Cooled oxygen also causes vascular dilation which stimulates the skin. It is however difficult, if not impossible, to safely and reliably supply cooled oxygen in a salon environment.

CN101670145 discloses a skin treatment device comprising a handset for including a dermal penetration device for contacting the skin surface in use and delivering a therapeutic or cosmetic agent. Also, US2011/092884 discloses a skin treatment device including an array of needles arrange to be inserted into the skin at a location where a stream of cold air is to be delivered.

KR20110106659 discloses a gun guide for accurately holding the insertion depth of a needle during a multi-hole procedure. The guide body can be provided with a cooling air supply part formed along the inside of the vacuum part while having a cooling air injection port through which cooling air can be provided from the outside and a plurality of cooling air injection holes leading to the treatment area.

It is an object of the present invention to address one or more of the above problems and to provide an alternative and/or improved skin treatment device.

The present invention is as described herein and in the claims.

According to the present invention there is provided a skin treatment device comprising a handset for supplying cooled oxygen-enriched air to the skin surface in use and a dermal penetration device for contacting the skin surface in use, the skin treatment device being capable of delivering the cooled oxygen-enriched air and a therapeutic or cosmetic agent onto the dermal penetration device (14).

Preferably, the therapeutic or cosmetic agent is contained in a cartridge in the handset.

Further preferably, the therapeutic or cosmetic agent is selected from the group consisting, but not limited to, any of the following: beauty care products and topical formulations.

In use, the cartridge may contain about 10ml to about 20ml of therapeutic or cosmetic agent.

Preferably, the cartridge contains about 13ml to about 17ml of therapeutic or cosmetic agent.

Further preferably, the cartridge contains about 15ml of therapeutic or cosmetic agent.

In use, the cooled oxygen-enriched air may have been passed through a peltier cooling module.

Preferably, the temperature of the cooled oxygen-enriched air can be controlled by altering the gaseous flow rate through the peltier cooling module and/or altering the electrical power being supplied to the peltier cooling module.

Further preferably, the peltier cooling module comprises two peltier units in thermal connection to the outer faces of a pair of thermally conductive plates.

In use, the inner faces of the thermally conductive plates may each contain a milled track or channel such that when the thermally conductive plates are secured together they define a contiguous and sealed channel through which the oxygen-enriched air can pass.

Preferably, the thermally conductive plates are thermally insulated from the surrounding ambient air.

Further, preferably, the temperature of the oxygen-enriched air is reduced by between about 8°C and about 16°C below the ambient air temperature.

In use, the temperature of the oxygen-enriched air may be cooled to about 20°C or below, about 18°C or below, about 16°C or below, about 14°C or below, about 12°C or below or about 10°C or below.

Preferably, the cartridge further comprises a coaxially positioned vent, and whereby any back flow of the therapeutic or cosmetic agent through the vent is prevented by a one-way exhaust valve.

Further preferably, the cartridge is retained inside an aperture in the handset via an interference fit and retaining clip.

In use, the dermal penetration device may be selected from the group consisting, but not limited to, any of the following: micro needle roller, dermal roller and needling device.

Preferably, the micro needle roller is retained in a removable cap, the cap partially enveloping the micro needle roller so that generally less than half of the micro needles situated around the outer circumference of the roller are exposed for contacting the skin surface in use.

Further preferably, the removable cap is disposed after one use.

In use, the handset may further comprise a nozzle in fluid connection with the cooled oxygen-enriched air, the nozzle also comprises a generally perpendicular connection to the therapeutic or cosmetic agent contained in the cartridge such that a venturi effect occurs through a constricted section of the nozzle.

Preferably, the therapeutic or cosmetic agent and the cooled oxygen-enriched air is sprayed onto the dermal penetration device.

Further preferably, the oxygen-enriched air is supplied from a first oxygen concentrator having an oxygen concentration of about 88% to about 98%, and a pressure flow from about 0.8bar to 1.8bar.

In use, a second oxygen concentrator may be provided having an oxygen concentration of about 75% to about 85% and a pressure flow from about 1.7bar to 2.7bar, the second oxygen concentrator supplying a secondary handset to supply cooled oxygen-enriched air the skin surface in use.

Preferably, each of the outer faces of the thermally conductive plates has an area of about 200cm2.

Further, there is disclosed a device comprising:
enriching means for generating oxygen-enriched air;
cooling means for cooling the oxygen-enriched air;
flexible conveying means for conveying the cooled oxygen-enriched air;
a handset connectable to the flexible conveying means, the handset comprises means for receiving a removable cartridge containing a therapeutic or cosmetic agent, a removable micro needle roller and a mixing nozzle through which the cooled oxygen-enriched air and therapeutic or cosmetic agent is sprayed onto the outer circumference of the micro needle roller for application to the skin surface in use.

It is believed that a skin treatment device in accordance with the present invention at least addresses the problems outlined above. The effect of the cooled, oxygen-enriched air passing over the treatment area serves to reduce the pain that is often associated with traditional dermal roller treatments. The cooled oxygen-enriched gaseous stream and cosmetic agent mixture is able to be driven into the skin through the micro punctures in the epidermis caused by the micro needles.

The use of cooled oxygen therefore enhances the therapeutic and/or cosmetic effect and also reduces pain and discomfort.

It will be obvious to those skilled in the art that variations of the present invention are possible and it is intended that the present invention may be used other than as specifically described herein.

Specific non-limiting embodiments of the invention will now be described by way of example only and with reference to the accompanying drawings, in which:
Figure 1 is a cross-sectional side view of a handset for supplying cooled oxygen-enriched air to the skin according to the present invention;
Figure 2 is a side perspective view from below of the handset as shown in Figure 1;
Figure 3 is a simplified schematic diagram illustrating how cooled, oxygen-enriched air is supplied to the handset;
Figure 4 shows how the oxygen-enriched air is passed through a peltier cooler in an expanded condition to provide a cooled gaseous stream to the skin; and
Figure 5 shows how the handset of Figure 1 can be used on the skin.

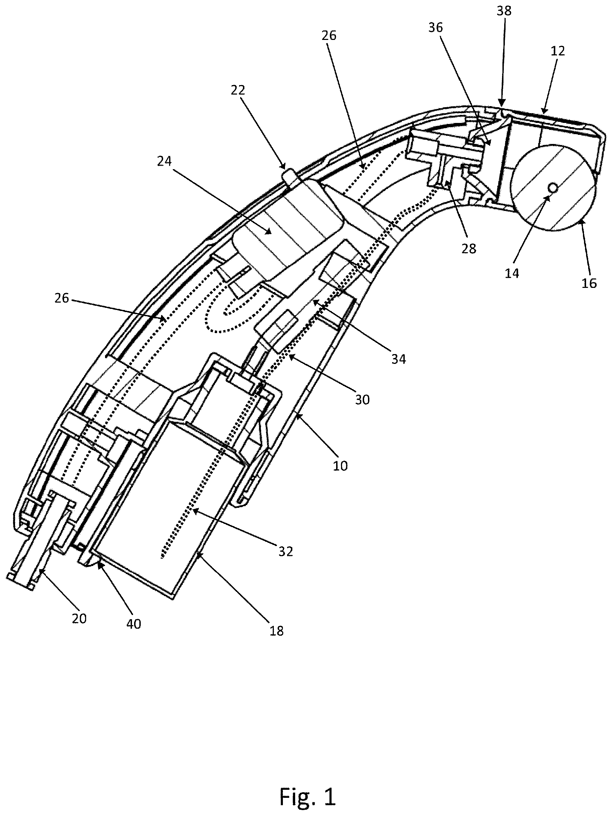

As can be seen from Figure 1, the handset 10 is provided as a generally elongate and ergonomically-shaped portion, having a generally circular cross-section. At one end of the handset 10 there is positioned a removable cap 12 which contains a roller 14 that is free to rotate on the skin. In a preferred embodiment, around the outer circumference 16 of the roller 14, an array of micro needles are positioned so that the roller 14 has the function of a dermal roller. The cap 12 partially envelops the roller 14 so that generally less than half of the micro needles arounds the outer circumference 16 of the roller 14 are exposed for allowing contact of the micro needles with the patient's skin. This has the advantage that the micro needles generally puncture the epidermis of the patient in a generally perpendicular manner and the micro needles are less susceptible to physical damage in use.

The cap 12 is releasably connected to the handset 10 by virtue of a push-fit type fitting 38 in the cap which meets with a respective channel around the periphery of the handset 10.

In use, the cap 12 containing the roller 14, is disposable after treatment of a patient.

The other end of the handset 10 is connected to cooled, oxygen-enriched air via a connector 20 which is connected to an oxygen-enriched air supply (not shown) via a flexible hose (not shown). Control of the cooled, oxygen-enriched air through the handset 10 is achieved by a sliding button 22 which controls valve 24. Sliding the button 22 to one end of its full movement opens valve 24 fully so that cooled, oxygen-enriched air flows from the connector 20, through the internal tubing 26, and a cooled gaseous stream is emitted or blown onto the roller 14 though an opening 36. Sliding the button 22 to the other end of its full movement closes valve 24 fully. Sliding the button 22 to a position somewhere between fully open and fully closed allows the operator to accurately control the flow of cooled, oxygen-enriched air to the skin of the patient.

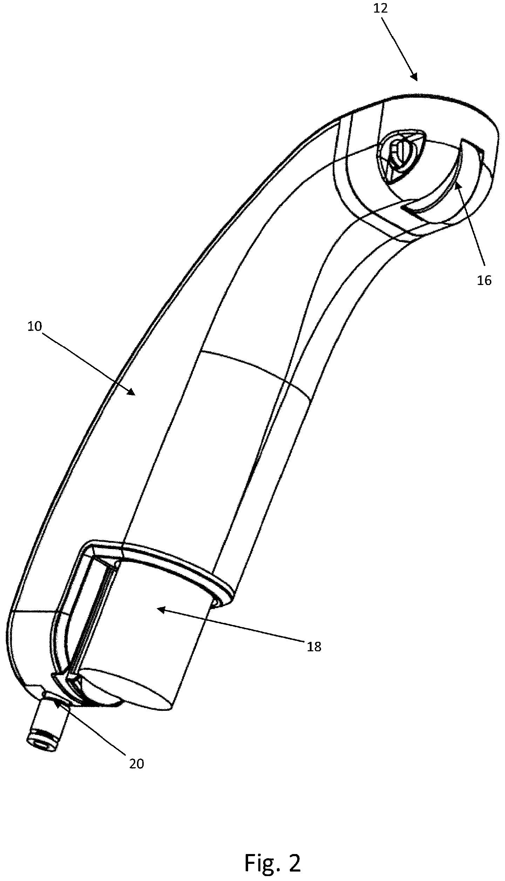

Figures 1 and 2 also show that a removable cartridge 18 can also be retained in the other end of the handset 10. The removable cartridge 18 contains a therapeutic or cosmetic agent or lotion to be applied to the skin. The cartridge 18 is retained inside an aperture in the handset 10 via an interference fit and retaining clip 40. A small bore conduit 32 draws the therapeutic or cosmetic agent from the cartridge 18 through flexible tubing 30. The therapeutic or cosmetic agent is conveyed along the internal tubing 30 to a nozzle 28 positioned at opening 36. The cartridge 18 also includes a coaxially positioned vent. Any back flow of the therapeutic or cosmetic agent through the vent which would otherwise occur, for example, if the handset was positioned upside-down on the patient is prevented by a one-way exhaust valve 34.

Whilst the cartridge 18 described herein in relation to Figure 1 connects by way of an interference fit, the skilled person will appreciate that any form of releasable connection to the handset 10 is possible, including, for example, connection by way of a screw thread, snap fit engagement, bayonet, quick release mechanism etc. The above list is in no way intended to be limiting and exhaustive.

As can be clearly seen from Figure 1, as cooled, oxygen-enriched air passes through the nozzle 28 a venturi effect occurs through the constricted section of the nozzle 28 which then sprays the therapeutic or cosmetic agent through the opening 36 with the cooled oxygen-enriched air.

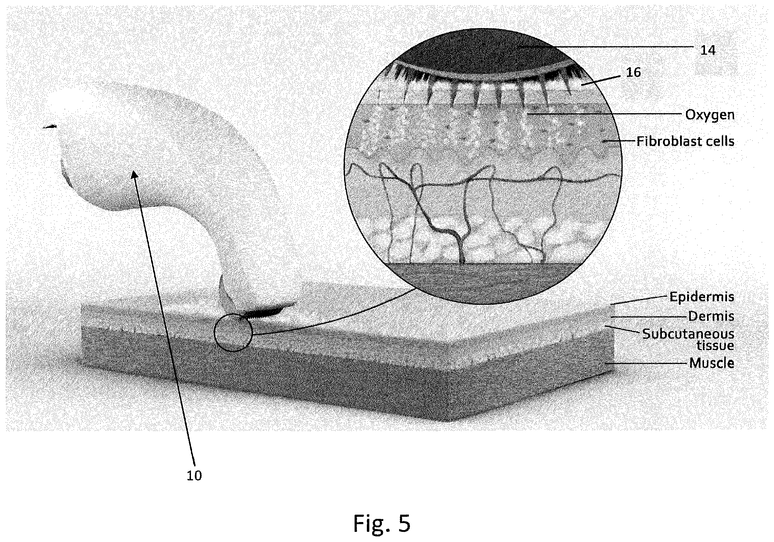

The cooled oxygen-enriched air and the cosmetic agent mixture is sprayed onto the outer circumference 16 of the roller 14 for application to the skin as the roller 14 is drawn over the patient's skin. The effect of the cooled, oxygen-enriched air passing over the treatment area serves to reduce the pain that is often associated with traditional dermal roller treatments. The cooled oxygen-enriched air and cosmetic agent mixture is then able to be driven into the skin through the micro punctures in the epidermis caused by the roller 14. This effect is illustrated in Figure 5.

In a preferred embodiment, the cartridge 18 contains 15ml of a therapeutic or cosmetic agent or lotion. This is in no way intended to be limiting as any number of different capacity cartridges 18 are envisaged. The volume of the cartridge 18 being dependent upon the area and type of skin being treated and the therapeutic or cosmetic agent or lotion being used.

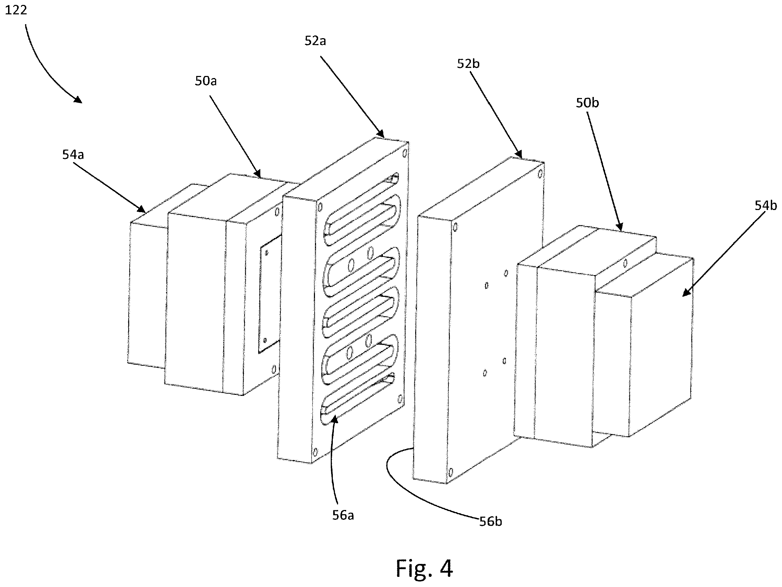

In a preferred embodiment, the temperature of the cooled, oxygen-enriched air is between 4°C and 12°C. The perception of the temperature felt by the skin depends on the ambient temperature. Therefore, the oxygen-enriched air is cooled by between 8°C and 16°C below the ambient temperature using a peltier cooling arrangement as described in detail in Figure 4. As well as the ambient temperature, the skilled person will appreciate that the slower the flow rate of the oxygen-enriched air through the peltier cooler, the greater the residence time in the cooler and the greater the reduction in temperature.

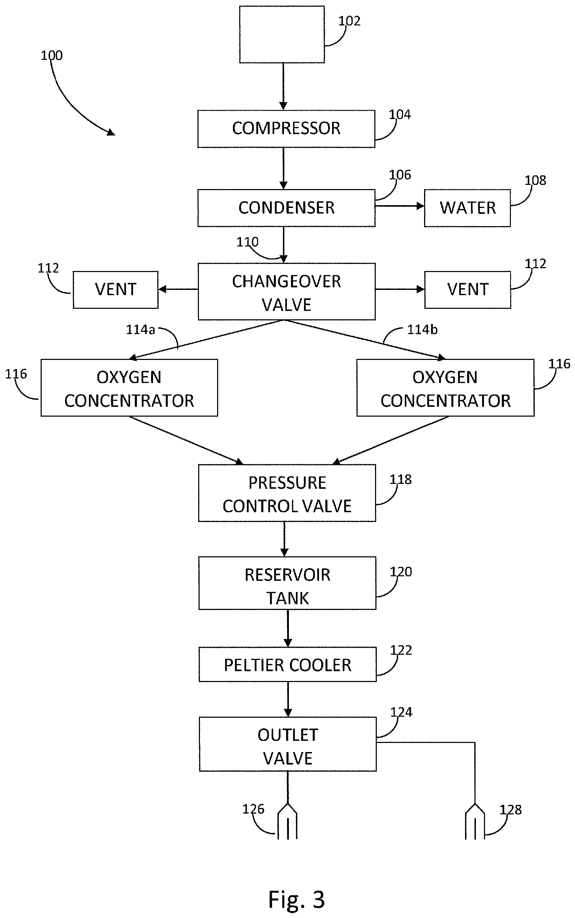

The cooled, oxygen-enriched air supplied to the handset 10 is produced using the system shown in Figure 3. This is identical to that described in GB 2 419 532 A. The present invention may also include the additional processing step of cooling the oxygen-enriched air using a peltier cooler arrangement 122.

In essence, the cooled, oxygen-enriched air system 100 draws air 102 into the system 100 via a compressor 104 that causes the circulation of the air throughout the system 100. The air is then passed on to a condenser 106 where the air is cooled and water vapour removed 108. Subsequently the air passes through a changeover valve 110 to one of two oxygen concentrator units 116a, 116b. Each oxygen concentrator unit 116a, 116b enriches the air so that it has a higher proportion of oxygen and each supplies oxygen-enriched air to an outlet valve 124 via a reservoir tank 120. The outlet valve 124 selectively couples the reservoir tank's 120 outlets to one of two gas outlets 126, 128 used to supply the oxygen-enriched air to the skin being treated. One gas outlet 126 supplies a higher volume of air and one gas outlet 128 supplies a higher proportion of oxygen.

The two gas outlets 126, 128 are used to supply oxygen-enriched air to the skin being treated. The handset 10 according to the present invention is connected to the high pressure outlet 126 and it is envisaged that a second separate handset (not shown) can be connected to the low pressure output 128 which could be a simple nozzle to supply oxygen to the skin for therapeutic and/or cosmetic benefit without any roller being attached. The advantages of having two separate handsets are that the operator can quickly and easily perform a transdermal infusion using handset 10, and then with a separate handset apply further cooled, oxygen-enriched air to the skin.

The first gas outlet 126 supplies cooled, oxygen-enriched air with an oxygen concentration of 93%. The flow rate through this outlet 126 is fixed so that such a percentage is always achieved at the expense of the pressure. The second gas outlet 128 supplies a gas which is approximately 80% oxygen but at a higher pressure than that produced by the other outlet 126.

Production of cooled, oxygen-enriched air is achieved by placing a peltier cooler 122 between the outlet of the reservoir tank 120 and the outlet valve 124. The oxygen-enriched air is cooled by between 8°C and 16°C below the ambient temperature using the peltier cooler 122, as shown in detail in Figure 4.

Figure 4 shows an expanded view of the peltier cooling module 122 according to the preferred embodiment. Two peltier units 50a, 50b are sandwiched to the outer faces of a pair of aluminium plates 52a, 52b. The inner faces of the aluminium plates 52a, 52b each containing a milled track or channel 56a, 56b such that when the aluminium plates 52a, 52b are secured together they define a contiguous and sealed channel through which oxygen-enriched air can pass. The contiguous and sealed channel defines a continuous cooling transfer path for the oxygen-enriched air to contact the surface of plates 52a, 52b which are, in turn, connected to the peltier modules 50a, 50b via their respective electrical supplies 54a, 54b. In a preferred embodiment, the area of each of the outer faces of the aluminium plates 52a, 52b is around 200cm².

As can be appreciated, the oxygen-enriched air is passed through the contiguous and sealed channel through an inlet and is removed at a corresponding outlet. To preserve the clarity of Figure 4, the respective inlet and outlet are not shown.

When assembled, the peltier cooling module 122 is covered with an insulating material to minimise cooling loss. In a preferred embodiment, the peltier cooling module 122 comprises two opposing peltier modules 50a, 50b. This is in no way intended to be limiting as any number of peltier devices or even alternative cooling techniques could be envisaged.

Various alterations and modifications may be made to the present invention without departing from the scope of the invention.

## Claims

1. A skin treatment device comprising a handset (10) for supplying cooled oxygen-enriched air to the skin surface in use and a dermal penetration device (14) for contacting the skin surface in use, the skin treatment device being capable of delivering the cooled oxygen-enriched air and a therapeutic or cosmetic agent onto the dermal penetration device (14).

2. The skin treatment device as claimed in claim 1, wherein the therapeutic or cosmetic agent is contained in a cartridge (18) in the handset (10); and/or optionally
wherein the therapeutic or cosmetic agent is selected from the group consisting, but not limited to, any of the following: beauty care products and topical formulations.

3. The skin treatment device as claimed in claim 2, wherein the cartridge (18) contains about 10ml to about 20ml of therapeutic or cosmetic agent; and/or optionally
wherein the cartridge (18) contains about 13ml to about 17ml of therapeutic or cosmetic agent; and/or optionally
wherein the cartridge (18) contains about 15ml of therapeutic or cosmetic agent; and/or optionally
wherein the cartridge (18) further comprises a coaxially positioned vent, and whereby any back flow of the therapeutic or cosmetic agent through the vent is prevented by a one-way exhaust valve; and/or optionally
wherein the cartridge (18) is retained inside an aperture in the handset via an interference fit and retaining clip (40); and/or optionally
wherein the handset further comprises a nozzle (28) in fluid connection with the cooled oxygen-enriched air, the nozzle (28) also comprises a generally perpendicular connection to the therapeutic or cosmetic agent contained in the cartridge such that a venturi effect occurs through a constricted section of the nozzle.

4. The skin treatment device as claimed in claim 1, wherein the cooled oxygen-enriched air has been passed through a peltier cooling module (122).

5. The skin treatment device as claimed in claim 4, wherein the temperature of the cooled oxygen-enriched air can be controlled by altering the gaseous flow rate through the peltier cooling module (122) and/or altering the electrical power being supplied to the peltier coaling module.

6. The skin treatment device as claimed in claims 4 or 5, wherein the peltier cooling module comprises two peltier units (50a, 50b) in thermal connection to the outer faces of a pair of thermally conductive plates (52a, 52b).

7. The skin treatment device as claimed in claim 6, wherein the inner faces of the thermally conductive plates (52a, 52b) each contain a milled track or channel such that when the thermally conductive plates are secured together they define a contiguous and sealed channel through which oxygen-enriched air can pass; and/or optionally
wherein the thermally conductive plates are thermally insulated from the surrounding ambient air; and/or optionally
wherein each of the outer faces of the thermally conductive plates has an areas of about 200cm².

8. The skin treatment device as claimed in claim 4, wherein the temperature of the oxygen-enriched air is reduced by between about 8°C and about 16°C below the ambient air temperature; and/or optionally
wherein the temperature of the oxygen-enriched air is cooled to about 20°C or below, about 18°C or below, about 16°C or below, about 14°C or below, about 12°C or below or about 10°C or below.

9. The skin treatment device as claimed in claim 1, wherein the dermal penetration device (14) is selected from any of the following: micro needle roller, dermal roller and needling device.

10. The skin treatment device as claimed in claim 9, wherein the dermal penetration device comprises a micro needle roller retained in a removable cap (12), the cap partially enveloping the micro needle roller so that generally less than half of the micro needles situated around the outer circumference of the roller are exposed for contacting the skin surface in use.

11. The skin treatment device as claimed in claim 10, wherein the removable cap (12) is disposed after one use.

12. The skin treatment device as claimed in claim 3, wherein the therapeutic or cosmetic agent and the cooled oxygen-enriched air is sprayed onto the dermal penetration device (14).

13. The skin treatment device as claimed in claim 4, wherein the oxygen-enriched air is supplied from a first oxygen concentrator having an oxygen concentration of about 88% to about 98%, and a pressure flow from about 0.8bar to 1.8bar.

14. The skin treatment device as claimed in claim 13, further comprising a second oxygen concentrator having an oxygen concentration of about 75% to about 85% and a pressure flow from about 1.7bar to 2.7bar, the second oxygen concentrator supplying a secondary handset to supply cooled oxygen-enriched air to the skin surface in use.

## Patentansprüche

1. Hautbehandlungsvorrichtung, umfassend ein Handgerät (10) zum Zuführen von gekühlter, mit Sauerstoff angereicherter Luft zur Hautoberfläche in Verwendung und eine dermale Penetrationsvorrichtung (14) zum Kontaktieren der Hautoberfläche in Verwendung, wobei die Hautbehandlungsvorrichtung in der Lage ist, die gekühlte, mit Sauerstoff angereicherte Luft und ein therapeutisches oder kosmetisches Mittel auf die dermale Penetrationsvorrichtung (14) abzugeben.

2. Hautbehandlungsvorrichtung nach Anspruch 1, wobei das therapeutische oder kosmetische Mittel in einer Patrone (18) in dem Handgerät (10) enthalten ist und/oder optional
wobei das therapeutische oder kosmetische Mittel aus der Gruppe bestehend aus einem des Folgenden, jedoch nicht darauf beschränkt, ausgewählt ist: Schönheitspflegeprodukten und topischen Formulierungen.

3. Hautbehandlungsvorrichtung nach Anspruch 2, wobei die Patrone (18) etwa 10 ml bis etwa 20 ml therapeutisches oder kosmetisches Mittel enthält und/oder optional
wobei die Patrone (18) etwa 13 ml bis etwa 17 ml therapeutisches oder kosmetisches Mittel enthält und/oder optional
wobei die Patrone (18) etwa 15 ml therapeutisches oder kosmetisches Mittel enthält und/oder optional
wobei die Patrone (18) ferner eine koaxial positionierte Lüftungsöffnung umfasst und wodurch jeglicher Rückfluss des therapeutischen oder kosmetischen Mittels durch die Lüftungsöffnung durch ein Einwegauslassventil verhindert wird und/oder optional
wobei die Patrone (18) innerhalb einer Öffnung in dem Handgerät über eine Presspassung und Halteklammer (40) gehalten wird und/oder optional
wobei das Handgerät ferner eine Düse (28) in Fluidverbindung mit der gekühlten, mit Sauerstoff angereicherten Luft umfasst, wobei die Düse (28) ebenfalls eine allgemein senkrechte Verbindung mit dem therapeutischen oder kosmetischen Mittel, das in der Patrone enthalten ist, derart umfasst, dass ein Venturi-Effekt durch einen verengten Abschnitt der Düse auftritt.

4. Hautbehandlungsvorrichtung nach Anspruch 1, wobei die gekühlte, mit Sauerstoff angereicherte Luft durch ein Peltier-Kühlmodul (122) geleitet worden ist.

5. Hautbehandlungsvorrichtung nach Anspruch 4, wobei die Temperatur der gekühlten, mit Sauerstoff angereicherten Luft durch Verändern der Gasdurchflussrate durch das Peltier-Kühlmodul (122) und/oder Verändern der elektrischen Leistung, die dem Peltier-Kühlmodul zugeführt wird, gesteuert werden kann.

6. Hautbehandlungsvorrichtung nach Ansprüchen 4 oder 5, wobei das Peltier-Kühlmodul zwei Peltier-Einheiten (50a, 50b) in thermischer Verbindung mit den Außenflächen eines Paars von Wärmeleitplatten (52a, 52b) umfasst.

7. Hautbehandlungsvorrichtung nach Anspruch 6, wobei die Innenflächen der Wärmeleitplatten (52a, 52b) jeweils eine eingefräste Spur oder einen eingefrästen Kanal derart enthalten, dass sie einen zusammenhängenden und abgedichteten Kanal definieren, durch den mit Sauerstoff angereicherte Luft strömen kann, wenn die Wärmeleitplatten aneinander befestigt sind, und/oder optional
wobei die Wärmeleitplatten thermisch von der Umgebungsluft isoliert sind und/oder optional
wobei jede der Außenflächen der Wärmeleitplatten einen Flächeninhalt von etwa 200 cm² aufweist.

8. Hautbehandlungsvorrichtung nach Anspruch 4, wobei die Temperatur der mit Sauerstoff angereicherten Luft um zwischen etwa 8 °C und etwa 16 °C unterhalb der Umgebungslufttemperatur gesenkt wird und/oder optional
wobei die Temperatur der mit Sauerstoff angereicherten Luft auf etwa 20 °C oder darunter, etwa 18 °C oder darunter, etwa 16 °C oder darunter, etwa 14 °C oder darunter, etwa 12 °C oder darunter oder etwa 10 °C oder darunter gekühlt wird.

9. Hautbehandlungsvorrichtung nach Anspruch 1, wobei die dermale Penetrationsvorrichtung (14) aus einem des Folgenden ausgewählt ist: Mikronadelroller, Dermaroller und Nadelungsvorrichtung.

10. Hautbehandlungsvorrichtung nach Anspruch 9, wobei die dermale Penetrationsvorrichtung einen Mikronadelroller umfasst, der in einer abnehmbaren Kappe (12) gehalten wird, wobei die Kappe den Mikronadelroller teilweise umschließt, sodass allgemein weniger als die Hälfte der Mikronadeln, die sich um den Außenumfang des Rollers herum befinden, zum Kontaktieren der Hautoberfläche in Verwendung freigelegt sind.

11. Hautbehandlungsvorrichtung nach Anspruch 10, wobei die abnehmbare Kappe (12) nach einer Verwendung entsorgt wird.

12. Hautbehandlungsvorrichtung nach Anspruch 3, wobei das therapeutische oder kosmetische Mittel und die gekühlte, mit Sauerstoff angereicherte Luft auf die dermale Penetrationsvorrichtung (14) gesprüht wird.

13. Hautbehandlungsvorrichtung nach Anspruch 4, wobei die mit Sauerstoff angereicherte Luft von einem ersten Sauerstoffkonzentrator mit einer Sauerstoffkonzentration von etwa 88 % bis etwa 98 % und einem Druckfluss von etwa 0,8 bar bis 1,8 bar zugeführt wird.

14. Hautbehandlungsvorrichtung nach Anspruch 13, ferner umfassend einen zweiten Sauerstoffkonzentrator mit einer Sauerstoffkonzentration von etwa 75 % bis etwa 85 % und einem Druckfluss von etwa 1,7 bar bis 2,7 bar, wobei der zweite Sauerstoffkonzentrator ein sekundäres Handgerät versorgt, um der Hautoberfläche in Verwendung gekühlte, mit Sauerstoff angereicherte Luft zuzuführen.

## Revendications

1. Dispositif de traitement de la peau comprenant un combiné (10) pour fournir de l'air enrichi en oxygène refroidi à la surface de la peau en cours d'utilisation et un dispositif de pénétration cutanée (14) pour entrer en contact avec la surface de la peau en cours d'utilisation, le dispositif de traitement de la peau étant capable de fournir de l'air enrichi en oxygène refroidi et un agent thérapeutique ou cosmétique dans le dispositif de pénétration cutanée (14).

2. Dispositif de traitement de la peau selon la revendication 1, dans lequel l'agent thérapeutique ou cosmétique est contenu dans une cartouche (18) dans le combiné (10) ; et / ou éventuellement
dans lequel l'agent thérapeutique ou cosmétique est choisi dans le groupe constitué, mais sans s'y limiter, par l'un quelconque des produits suivants : des produits de soins de beauté et formulations topiques.

3. Dispositif de traitement de la peau selon la revendication 2, dans lequel la cartouche (18) contient environ 10 ml à environ 20 ml d'agent thérapeutique ou cosmétique ; et / ou éventuellement
dans lequel la cartouche (18) contient environ 13 ml à environ 17 ml d'agent thérapeutique ou cosmétique ; et / ou
éventuellement dans lequel la cartouche (18) contient environ 15 ml d'agent thérapeutique ou cosmétique ; et / ou
éventuellement dans lequel la cartouche (18) comprend en outre un évent positionné coaxialement, et grâce à quoi tout reflux de l'agent thérapeutique ou cosmétique à travers l'évent est empêché par une soupape d'échappement unidirectionnelle ; et / ou
éventuellement dans lequel la cartouche (18) est retenue à l'intérieur d'une ouverture dans le combiné via un ajustement serré et un clip de retenue (40) ; et / ou
éventuellement dans lequel le combiné comprend en outre une buse (28) en connexion fluidique avec l'air enrichi en oxygène refroidi, la buse (28) comprend également une connexion généralement perpendiculaire à l'agent thérapeutique ou cosmétique contenu dans la cartouche de telle sorte qu'un effet venturi se produit à travers une section rétrécie de la buse.

4. Dispositif de traitement de la peau selon la revendication 1, dans lequel l'air enrichi en oxygène refroidi est passé à travers un module de refroidissement Peltier (122).

5. Dispositif de traitement de la peau selon la revendication 4, dans lequel la température de l'air enrichi en oxygène refroidi peut être contrôlée en modifiant le débit gazeux à travers le module de refroidissement Peltier (122) et / ou en modifiant la puissance électrique fournie au module de refroidissement Peltier.

6. Dispositif de traitement de la peau selon la revendication 4 ou 5, dans lequel le module de refroidissement Peltier comprend deux unités Peltier (50a, 50b) en liaison thermique avec les faces externes d'une paire de plaques thermoconductrices (52a, 52b).

7. Dispositif de traitement de la peau selon la revendication 6, dans lequel les faces internes des plaques thermoconductrices (52a, 52b) contiennent chacune une piste ou un canal fraisé de telle sorte que lorsque les plaques thermoconductrices sont fixées ensemble, elles définissent un canal contigu et étanche à travers lequel l'air enrichi en oxygène peut passer ; et / ou éventuellement
dans lequel les plaques thermoconductrices sont isolées thermiquement de l'air ambiant environnant ; et / ou éventuellement
dans lequel chacune des faces externes des plaques thermoconductrices a une superficie d'environ 200 cm².

8. Dispositif de traitement de la peau selon la revendication 4, dans lequel la température de l'air enrichi en oxygène est réduite d'environ 8 ° C à environ 16 ° C en dessous de la température de l'air ambiant ; et / ou éventuellement
dans lequel la température de l'air enrichi en oxygène est refroidie à environ 20 ° C ou moins, environ 18 ° C ou moins, environ 16 ° C ou moins, environ 14 ° C ou moins, environ 12 ° C ou moins ou environ 10 ° C ou moins.

9. Dispositif de traitement de la peau selon la revendication 1, dans lequel le dispositif de pénétration cutanée (14) est choisi parmi l'un quelconque des éléments suivants : un rouleau à micro-aiguilles, un rouleau cutané et un dispositif d'aiguilletage.

10. Dispositif de traitement de la peau selon la revendication 9, **caractérisé en ce que** le dispositif de pénétration cutanée comprend un rouleau à micro-aiguilles retenu dans un capuchon amovible (12), le capuchon enveloppant partiellement le rouleau à micro-aiguilles de sorte que généralement moins de la moitié des micro-aiguilles se situant autour de la circonférence externe du rouleau sont exposées pour entrer en contact avec la surface de la peau en cours d'utilisation.

11. Dispositif de traitement de la peau selon la revendication 10, dans lequel le capuchon amovible (12) est disposé après une utilisation.

12. Dispositif de traitement de la peau selon la revendication 3, **caractérisé en ce que** l'agent thérapeutique ou cosmétique et l'air enrichi en oxygène refroidi sont pulvérisés sur le dispositif de pénétration cutanée (14).

13. Dispositif de traitement de la peau selon la revendication 4, dans lequel l'air enrichi en oxygène est fourni à partir d'un premier concentrateur d'oxygène ayant une concentration en oxygène d'environ 88% à environ 98%, et un débit de pression d'environ 0,8 bar à 1,8 bar.

14. Dispositif de traitement de la peau selon la revendication 13, comprenant en outre un second concentrateur d'oxygène ayant une concentration en oxygène d'environ 75% à environ 85% et un débit de pression d'environ 1,7 bar à 2,7 bar, le second concentrateur d'oxygène alimentant un combiné secondaire pour fournir de l'air enrichi en oxygène refroidi à la surface de la peau en cours d'utilisation.
